(19) Europäisches Patentamt
European Patent Office
Office européen des brevets

(11) Numéro de publication: **0 006 789**
**A1**

(12) **DEMANDE DE BREVET EUROPEEN**

(21) Numéro de dépôt: **79400375.6**

(22) Date de dépôt: **11.06.79**

(51) Int. Cl.[3]: **C 07 C 149/40**, C 07 C 59/70, C 07 C 59/68, A 61 K 31/19, C 07 C 51/367, C 07 C 148/00

(30) Priorité: **14.06.78 FR 7817814**

(43) Date de publication de la demande: **09.01.80 Bulletin 80/1**

(84) Etats contractants désignés: **AT DE FR IT NL SE**

(71) Demandeur: **Société Anonyme dite: HEXACHIMIE, 128, rue Danton, F-92500 Rueil-Malmaison (FR)**

(72) Inventeur: **Cognacq, Jean-Claude Gaston, 22, rue du Buat, F-78580 Maule (FR)**
Inventeur: **Lacrampe, Jean Fernand, 4, rue Edith Cavell, F-92400 Courbevoie (FR)**

(74) Mandataire: **de Haas, Michel et al, Cabinet Beau de Lomenie 55 rue d'Amsterdam, F-75000 Paris 8ème (FR)**

(54) **Composés bis (aryloxyalcanecarboxyliques), leur préparation et leur utilisation en thérapeutique.**

(57) Composés de formule:

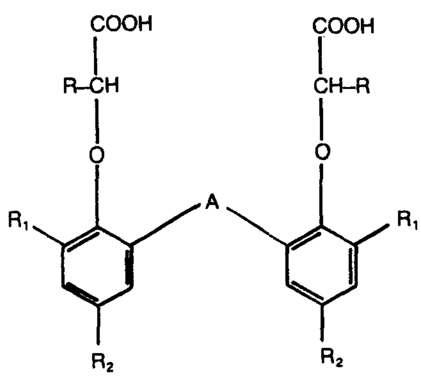

dans laquelle
- A est le groupement méthylène, le soufre, ou un pont disulfure,
- R est l'hydrogène ou le groupement méthyle,
- $R_1$ est un groupement alkoyle de 1 à 4 atomes de carbone, linéaire ou ramifié, et
- $R_2$ est un groupe alkoyle de 1 à 4 atomes de carbone, ou un atome d'halogène, ou un groupement alkoyloxy, et leurs sels pharmaceutiquement acceptables.

Préparation de ces composés à partir des phénols correspondants et leur utilisation thérapeutique.

EP 0 006 789 A1

Nouveaux composés bis(aryloxycarboxyliques), leur préparation et leur utilisation en thérapeutique.

La présente invention se rapporte à de nouveaux composés bis(aryloxycarboxyliques), à leur préparation et à leur utilisation, notamment en thérapeutique.

Ces nouveaux composés répondent à la formule générale (I) :

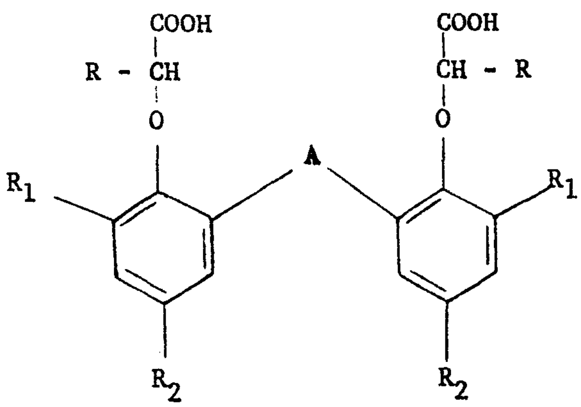

(I)

dans laquelle

- A est le groupement méthylène, le soufre, ou un pont disulfure,
- R est l'hydrogène ou le groupement méthyle,
- $R_1$ est un groupement alkoyle de 1 à 4 atomes de carbone, linéaire ou ramifié, et
- $R_2$ est un groupement alkoyle de 1 à 4 atomes de carbone, avantageusement le groupement méthyle, ou un atome d'halogène, avantageusement le chlore, ou un groupement alkoyloxy, avantageusement le groupement méthoxy.

La présente invention a trait également à un procédé de préparation des composés (I), selon lequel on fait réagir sur un phénol (II) :

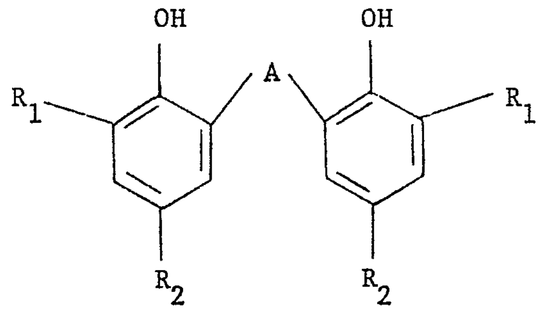

(II)

dans lequel A, $R_1$ et $R_2$ ont la signification ci-dessus, 2 équivalents d'un agent de sodation qui peut être l'hydrure de sodium, le méthylate de sodium, l'éthylate de sodium, etc., dans un solvant donneur d'électrons tel que le diméthylformamide, le diméthylsulfoxyde, etc., à une température d'environ 20-40°C, puis on fait réagir sur le dérivé sodé ainsi obtenu deux équivalents d'un dérivé halogéné (III) :

$$X - \underset{\displaystyle R}{\underset{|}{CH}} - COOR_3 \qquad \text{(III)}$$

dans lequel

- X est un atome d'halogène, avantageusement le brome,
- R a la signification ci-dessus,
- $R_3$ est un groupement alcoyle inférieur, avantageusement le radical méthyle ou le radical éthyle,

à une température d'environ 50-90°C, de préférence 70°C. Un traitement ultérieur par la soude ou la potasse en milieu alcoolique (méthanol ou éthanol) au reflux dudit solvant conduit aux composés (I).

Les composés de formule (I) jouissent de propriétés pharmacologiques qui les rendent précieux en thérapeutique. Ils sont notamment actifs dans le traitement des hyperlipidémies athérogènes.

Les exemples non limitatifs suivants sont donnés pour illustrer l'invention.

<u>Exemple 1 :</u>

<u>Sulfure de bis(carboxyméthoxy-2, t-butyl-3, méthyl-5 phényle) formule (I) avec : R = H ; $R_1$ = t-butyle ; $R_2$ = $CH_3$ ; et A = S.</u>

A une solution de 14,3 g (0,04 mole) de thio-2,2'-bis(méthyl-4-tertiobutyl-6-phénol) dans 100 ml de diméthylformamide anhydre, on ajoute, en plusieurs fois, 4,5 g d'hydrure de sodium à 50% dans l'huile végétale, et on maintient une bonne agitation pendant 1 heure à 40°C.

On ajoute ensuite, lentement, 9,8 g (0,08 mole) de chloroacétate d'éthyle : la réaction est exothermique. La solution est agitée 1 heure à température ambiante puis 5 heures à 50°C et elle est ensuite versée sur 250 ml d'eau.

On extrait à l'éther, sèche sur sulfate de magnésium, et évapore le solvant sous vide.

Le résidu obtenu (18,4 g) est traité au reflux pendant 7 heures par 3 g (0,075 mole) de soude en pastilles dans 150 ml d'éthanol.

Puis l'éthanol est évaporé. Le résidu est repris à l'eau et la solution obtenue est extraite à l'éther.

La phase aqueuse est ensuite acidifiée à pH 1 et extraite à l'éther. La phase éthérée est séchée sur sulfate de magnésium et évaporée ; on obtient 7,2 g de résidu que l'on recristallise dans le cyclohexane, pour obtenir 5,1 g de produit attendu fondant à 207°C.

Exemple 2 :

Sulfure de bis(α-carboxyéthoxy-2, t-butyl-3, méthyl-5 phényle) formule (I) avec : R = $CH_3$ ; $R_1$ = t-butyle ; $R_2$ = $CH_3$ ; et A = S.

Le mode opératoire est identique à celui de l'exemple précédent, mais à partir de 21,5 g (0,06 mole) de thio-2,2' bis(méthyl-4, tertiobutyl-6 phénol), 6,8 g d'hydrure de sodium à 50% dans 150 ml de diméthylformamide anhydre, et 15,7 ml de bromo-2 propionate d'éthyle.

Le résidu d'évaporation obtenu (29,2 g) est traité par 5 g de soude en pastilles dans 150 ml d'éthanol.

On isole 23,9 g de produit brut que l'on recristallise dans le pentane. On obtient 12,2 g de produit attendu fondant à 149°C.

Exemple 3 :

Sulfure de bis(α-carboxyéthoxy-2, méthyl-3, chloro-5 phényle) formule (I) avec : R = $CH_3$ ; $R_1$ = $CH_3$ ; $R_2$ = Cl ; et A = S.

Le mode opératoire est identique à celui de l'exemple 1, mais à partir de 20,4 g (0,065 mole) de thio-2,2' bis-(chloro-4 méthyl-6 phénol), 7,4 g d'hydrure de sodium à 50% dans 150 ml de diméthylformamide anhydre, et 17 ml de bromo-2 propionate d'éthyle.

Le résidu d'évaporation obtenu (21,6 g) est traité par 5 g de soude en pastilles dans 250 ml d'éthanol.

On isole 16,1 g de produit brut que l'on recristallise dans le cyclohexane. On obtient 11,6 g de composé attendu fondant à 135°C.

Exemple 4 :

(di[α-carboxyéthoxy]2,2', diméthyl-3,3', dichloro-5,5') diphénylméthane.

formule (I) avec : R = $CH_3$ ; $R_1$ = $CH_3$ ; $R_2$ = Cl et A = $CH_2$

Le mode opératoire est identique à celui de l'exemple 1, mais à partir de 11,8 g (0,04 mole) de méthylène-2,2' bis-(chloro-4 méthyl-6 phénol), 4,2 g d'hydrure de sodium à 50%, dans 100 ml de diméthylformamide anhydre et 12,6 ml de bromo-2 propionate d'éthyle.

Le résidu d'évaporation obtenu (18 g) est traité par 5 g de soude en pastilles dans 50 ml d'éthanol.

On isole 8,3 g de produit obtenu attendu, recristallisé dans un mélange cyclohexane-éther isopropylique (90/10), fondant à 178°C.

L'activité hypocholestérolémiante est recherchée chez des rats mâles Sprague Dawley pesant 130 g. Les produits étudiés sont administrés par voie orale pendant quatre jours à la dose journalière de 250 mg/kg.

Au cinquième jour, les animaux sont sacrifiés et il est procédé à un dosage de la cholestérolémie selon la méthode de Watson.

L'activité du produit connu appelé "clofibrate" a été étudiée sur ce test dans les mêmes conditions.

Le tableau ci-après donne les moyenne et écart type à la moyenne des cholestérolémies obtenus après traitement.

| lots | Nombre d'animaux | cholestérolémie $gl^{-1}$<br>- moyenne<br>- écart type à la moyenne |
|---|---|---|
| Témoins | 10 | 1,113<br>0,037 |
| Composé de l'exemple 1 | 10 | 0,847<br>0,053 |
| Composé de l'exemple 2 | 10 | 0,693<br>0,027 |
| Clofibrate | 10 | 0,854<br>0,053 |

Les études de toxicité faites chez le rat Sprague Dawley après administration orale ont permis de déterminer une dose létale zéro supérieure à 1 600 mg/kg pour les deux produits étudiés ci-dessus.

En conclusion, les composés selon l'invention possèdent une activité hypocholestérolémiante intéressante, le rapport activité/toxicité permettant une utilisation thérapeutique dans le traitement des hyperlipidémies athérogènes, sous forme de gélules, aux doses unitaires de 150 à 250 mg par prise, à raison de 4 prises par jour pour un être humain adulte d'environ 70 kg.

Bien entendu, l'invention concerne également les sels pharmaceutiquement acceptables des composés I, obtenus de manière classique.

# R E V E N D I C A T I O N S

1. Nouveaux composés, caractérisés en ce qu'ils répondent à la formule :

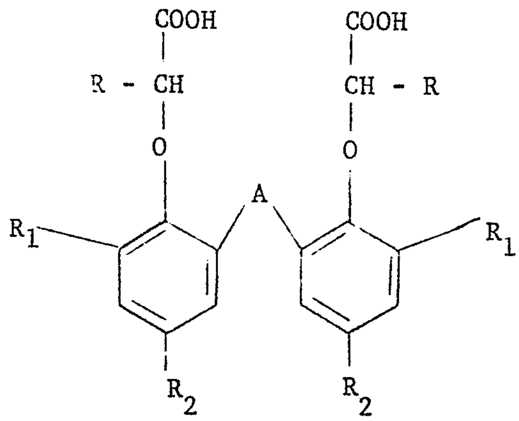

(I)

dans laquelle

- A est le groupement méthylène, le soufre, ou un pont disulfure,
- R est l'hydrogène ou le groupement méthyle,
- $R_1$ est un groupement alkoyle de 1 à 4 atomes de carbone, linéaire ou ramifié, et
- $R_2$ est un groupe alkoyle de 1 à 4 atomes de carbone, ou un atome d'halogène, ou un groupement alkoyloxy, et leurs sels pharmaceutiquement acceptables.

2. Nouveaux composés selon la revendication 1, caractérisés en ce que $R_2$ est de préférence choisi parmi le groupement méthyle, l'atome de chlore et le groupement méthoxy.

3. Nouveaux composés selon la revendication 1, caractérisés en ce qu'ils sont choisis parmi les composés suivants :

- sulfure de bis(carboxyméthoxy-2, t-butyl-3, méthyl-5 phényle),
- sulfure de bis(α-carboxyéthoxy-2, t-butyl-3, méthyl-5 phényle),
- sulfure de bis(α-carboxyéthoxy-2, méthyl-3, chloro-5 phényle),
- (di[α-carboxyéthoxy]-2,2', diméthyl-3,3', dichloro-5,5') diphénylméthane.

4. Procédé de préparation des composés selon la revendication 1, caractérisé en ce que l'on fait réagir un phénol (II) :

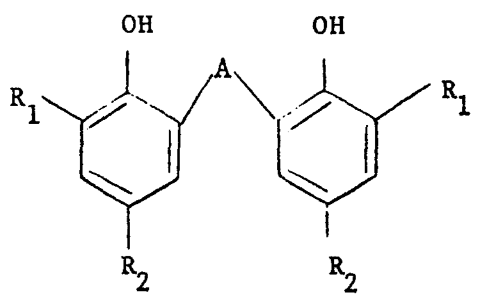

(II)

dans lequel :

A, $R_1$ et $R_2$ sont tels que définis dans la revendication 1, sur deux équivalents d'un agent de sodation, dans un solvant donneur d'électrons, à environ 20-40°C, après quoi on fait réagir le dérivé sodé ainsi obtenu sur deux équivalents d'un dérivé halogéné (III)

$$X - \underset{\displaystyle R}{\underset{|}{CH}} - COOR_3 \qquad (III)$$

dans lequel

- X est un atome d'halogène, de préférence le brome,
- R est un atome d'hydrogène ou un groupement méthyle, et
- $R_3$ est un groupement alcoyle inférieur, de préférence le groupe méthyle ou éthyle, à environ 50-90°C, de préférence 70°C, avant de faire réagir le produit obtenu sur la soude ou la potasse en milieu alcoolique, de préférence éthanol ou méthanol, pour obtenir les composés de formule (I).

5. Procédé selon la revendication 4, caractérisé en ce que l'agent de sodation est choisi parmi l'hydrure de sodium, le méthylate de sodium, et l'éthylate de sodium.

6. Procédé selon la revendication 4 ou 5, caractérisé en ce que le solvant est le diméthylformamide ou le diméthylsulfoxyde.

7. Nouveaux médicaments, caractérisés en ce qu'ils consistent en les nouveaux composés selon l'une quelconque des revendications 1 à 3 et leurs sels pharceutiquement acceptables.

8. Nouveaux médicaments selon la revendication 7 associés à des excipients pharmaceutiquement acceptables.

9. Nouveaux médicaments selon la revendication 7 ou 8, conditionnés pour l'administration par voie orale, en gélules, de 150 à 250 mg de principe actif par prise, la dose journalière étant de 0,6 à 1 g de principe actif, en 4 prises, pour un adulte.

0006789

Office européen des brevets

# RAPPORT DE RECHERCHE EUROPEENNE

Numéro de la demande

EP 79 40 0375

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| A | FR - A - 2 233 043 (BRISTOL-MYERS CO.)<br>* Page 10, lignes 2-3, ligne 37 - page 11, ligne 2 *<br>---- | 1,2,7 | C 07 C 149/32<br>C 07 C 59/25<br>A 61 K 31/19 |
| | | | **DOMAINES TECHNIQUES RECHERCHES (Int. Cl.³)** |
| | | | C 07 C 149/32<br>C 07 C 59/25<br>A 61 K 31/19 |
| | | | **CATEGORIE DES DOCUMENTS CITES** |
| | | | X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons |
| | Le présent rapport de recherche a été établi pour toutes les revendications | | &: membre de la même famille, document correspondant |

| Lieu de la recherche | Date d'achèvement de la recherche | Examinateur |
|---|---|---|
| La Haye | 04-09-1979 | SUTER |